# EUROPEAN PATENT SPECIFICATION

(11) **EP 3 028 040 B1**
(45) Date of publication and mention of the grant of the patent: **21.03.2018**
(21) Application number: 14750947.5
(22) Date of filing: 31.07.2014
(51) Int. Cl.: G01N 33/50

(54) **METHOD OF COLLECTING AND QUANTIFYING MELANIN IN SKIN**
VERFAHREN ZUR ERFASSUNG UND QUANTIFIZIERUNG VON MELANIN IN DER HAUT
PROCÉDÉ DE RECUEIL ET DE QUANTIFICATION DE LA MÉLANINE PRÉSENTE DANS LA PEAU

(30) Priority: 01.08.2013 US 201313957278
(43) Date of publication of application: 08.06.2016
(73) Proprietor: The Procter & Gamble Company, Cincinnati, OH 45202 (US)
(72) Inventor: WEHMEYER, Kenneth, Robert, Cincinnati, Ohio 45202 (US); OSORIO, Karen, Cincinnati, Ohio 45202 (US); STOFFOLANO, Peter, Joseph, Cincinnati, Ohio 45202 (US)
(74) Representative: Engisch, Gautier
(86) International application number: PCT/US2014/049142
(87) International publication number: WO 2015/017654

(56) References cited:
- JP-A- 2012 211 886
- KR-A- 20070 098 073
- S. COMMO ET AL: "Age-dependent changes in eumelanin composition in hairs of various ethnic origins", INTERNATIONAL JOURNAL OF COSMETIC SCIENCE, vol. 34, no. 1, 22 November 2011 (2011-11-22), pages 102-107, XP055148126, ISSN: 0142-5463, DOI: 10.1111/j.1468-2494.2011.00691.x
- PEARSE A ET AL: "Changes in melanin content of skin after UV irradiation", JOURNAL OF DERMATOLOGICAL SCIENCE, ELSEVIER SCIENCE PUBLISHERS, SHANNON, IE, vol. 6, no. 1, 1 August 1993 (1993-08-01), page 60, XP027341543, ISSN: 0923-1811 [retrieved on 1993-08-01]
- ANDO N ET AL: "The incidence of nuclear caps in dyschromia", JOURNAL OF DERMATOLOGICAL SCIENCE, ELSEVIER SCIENCE PUBLISHERS, SHANNON, IE, vol. 6, no. 1, 1 August 1993 (1993-08-01), page 60, XP027341542, ISSN: 0923-1811 [retrieved on 1993-08-01]
- ROGIERS V ET AL: "The potential use of non-invasive methods in the safety assessment of cosmetic products: The report and recommendations of an ECVAM/EEMCO Workshop (ECVAM Workshop 36)", ATLA. ALTERNATIVES TO LABORATORY ANIMALS, LONDON, GB, vol. 27, no. 4, 1 January 1999 (1999-01-01), pages 515-537, XP008139430, ISSN: 0261-1929

## Description

### FIELD

The method herein is directed, generally, to quantifying the amount of melanin present in skin. More specifically, the method is directed to noninvasively collecting skin cells and obtaining a derivative of melanin from the skin cells, which can be correlated to the amount of melanin present in the skin cells.

### BACKGROUND

Skin is a remarkable organ that includes multiple specialized tissues working together to provide a first line of defense against environmental insults. However, over time, intrinsic and extrinsic aging factors can damage skin tissues and may cause the skin to appear aged and/or discolored. For example, UV irradiation from the sun is known to cause skin aging, which is sometimes referred to as "photoaging". Unlike chronological aging, which depends solely on the passage of time, photoaging is a function of the degree to which one is exposed to the sun over a lifetime. The photoaging process is cumulative, with UV irradiation invoking a complex sequence of molecular responses that progressively damage the skin and may result in fine lines and wrinkles, freckles, lentigines, hyperpigmentation, hypopigmentation, and uneven tone. Some people find the appearance of skin discoloration undesirable. As a result, a multitude of cosmetic products have been developed to treat a wide variety of perceived skin flaws associated with aging. But many of these cosmetic products simply conceal flaws, for example, by covering the discolored skin portion or blending it to match the color of nearby skin rather than addressing the underlying cause of the discoloration. Thus, it would be desirable to reduce or eliminate undesired discoloration rather than simply hiding it or blending it in to the surrounding skin. However, the biological processes underlying skin discoloration are complex and not entirely understood.

One cause of skin discoloration is the overproduction of melanin in skin. Melanin is a ubiquitous natural pigment found in most organisms. The most common biological melanin is eumelanin, which is a brown-black polymer of dihydroxyindole carboxylic acids and their reduced forms. Another common form of melanin is pheomelanin, which is a cysteine-containing, yellow-red-brown polymer of benzothiazine units largely responsible for red hair and freckles. Melanin is produced by a complex set of reactions within a melanocyte involving, at a basic level, the interaction of the enzyme tyrosinase and the amino acid L-tyrosine. Tyrosinase catalyzes the conversion of L-tyrosine to DOPA (L-3,4-dihydroxyphenylalanine) and of DOPA to dopaquinone. Dopaquinone undergoes further conversion to form melanin. Melanin aggregates in organelles known as the melanosomes which are transferred to keratinocytes along slender filaments of the melanocyte known as dendrites. The production of tyrosinase and its activity determine, in part, the amount of melanin produced. The amount and the type of melanin transferred to the keratinocytes determine, for their part, the degree of visual pigmentation of human skin. In young skin, melanin is evenly distributed, and melanocyte activity is low, restricted to the production of constitutive pigmentation only. In aging skin, overzealous melanogenesis production and subsequent melanin transport can eventually create permanent local discoloration with sufficient size and contrast to appear as age spots (lentigines) or as diffuse hyperpigmentation or an uneven skin tone.

Because melanin production, especially eumelanin is believed to be linked to a variety of skin discoloration conditions, it may be desirable to quantify the amount of eumelanin present in a skin tissue sample or a particular portion of skin in order to compare different skin condition phenotypes according to the amount of the melanin present in the discolored portion of skin. But attempting to directly quantify the amount of eumelanin present in a sample such as a skin tissue sample is difficult because the chemical structure of eumelanin is not precisely known. In addition, the chemical properties of melanins, such as their insolubility over a broad range of pH, the heterogeneity in their structural features, and the lack of methods to split melanin polymers into their monomer units make direct measurement difficult. Further, many conventional methods for quantifying melanin require the isolation of melanin from a tissue sample and are not able to distinguish between eumelanin and pheomelanin.

In order to distinguish the amount of eumelanin present in a sample from the amount of pheomelanin, a relatively rapid method of quantifying melanin in tissue samples was introduced by Wakamatsu, et al., which made the isolation of melanin pigments unnecessary (Wakamatsu, et al., "Advanced Chemical Methods in Melanin Determination." PIGMENT CELL RES 15: 174-183. 2002). The method introduced by Wakamatsu is based on the formation of pyrrole-2,3,5-tricarboxylic acid ("PTCA") by permanganate oxidation of eumelanin and the formation of aminohydroxyphenylalanine ("AHP") isomers by hydriodic acid reductive hydrolysis of pheomelanin. The PTCA and AHP degradation products are determined by HPLC. PTCA is quantified with UV detection and AHP is quantified with electrochemical detection. While the Wakamatsu method improved the speed to quantify melanin and the ability to distinguish between the amount of eumelanin and pheomelanin, the Wakamatsu method relies on the use of live skin cells to determine melanin amount, which are commonly obtained from a donor using a conventional biopsy procedure.

A biopsy generally involves invasively removing a portion of tissue to be tested from a donor. The drawbacks associated with collecting biological material using a biopsy (or other invasive procedures) include increased risk of mortality and morbidity, undesirable impact of the state of the skin, difficulty related to execution on a large population, discomfort to the participant, and increased risk of infection. While a biopsy may provide a means for collecting biological samples for testing, it would be desirable to provide a noninvasive way to obtain a biological sample for quantifying melanin content in skin.

KR 20070098073 and JP 2012211886 disclose methods involving the collection and quantification of melanin in skin.

### SUMMARY

In order to provide a solution to the problems above, there is provided a method as in the claims.

### BRIEF DESCRIPTION OF THE DRAWINGS

FIGS. 1A, 1B and 1C are side views of an example of a sample collection device.
FIG. 2 shows areas of a face that may be sampled according to the present method.
FIG. 3 shows an area near the back of the ear that may be sampled according to the present method.
FIG. 4 illustrates the amount of PTCA obtained from samples collected from various areas of the face.
FIGS. 5A, 5B and 5C illustrate the PTCA levels of 3 different portions of skin from 5 test subjects.
FIG. 6 illustrates the amount of PTCA obtained from samples of different portions of skin from the same test subject.
FIGS 7A and 7B show Fontana Masson stained skin samples from two different test subjects.
FIG 8 illustrates the amount of PTCA obtained from samples collected from the same individuals as FIGS. 7A and 7B.

### DETAILED DESCRIPTION OF THE INVENTION

All percentages are by weight, based on the weight of the composition, unless otherwise specified. All ratios are weight ratios, unless specifically stated otherwise. All weights, as they pertain to listed ingredients, are based on the active level and, therefore, do not include carriers or by-products that may be included in commercially available materials. All numeric ranges are inclusive of narrower ranges; delineated upper and lower range limits are interchangeable to create further ranges not explicitly delineated. The number of significant digits conveys neither limitation on the indicated amounts nor on the accuracy of the measurements. All measurements are understood to be made at about 25° C and at ambient conditions, where "ambient conditions" means conditions under about one atmosphere of pressure and at about 50% relative humidity.

Except as otherwise noted, the articles "a", "an", and "the" mean "one or more." As used herein, "comprising" means that other steps and other ingredients which do not affect the end result can be added, and encompasses the terms "consisting of' and "consisting essentially of". The compositions and methods/processes of the present invention can comprise, consist of, and consist essentially of the essential elements and limitations of the invention described herein, as well as any of the additional or optional ingredients, components, steps, or limitations described herein.

"Derivative of melanin" means a compound that is derived from melanin (i.e. eumelanin or pheomelanin) by a chemical or physical process. The suitable melanin derivative for use in the present method should be capable of being correlated to the amount of melanin present in the sample from which it was obtained, and is pyrrole-2,3,5-tricarboxylic acid (eumelanin).

"Fitzpatrick Scale" refers to a commonly known numerical classification system for the color of skin, which was originally developed in 1975 by Thomas B. Fitzpatrick, a Harvard dermatologist, as a way to classify the response of different types of skin to UV light. The Fitzpatrick Scale classifies skin into six different types as follows: Type I (scores 0-7) Light, pale white - always burns, never tans; Type II (scores 8-16) White, fair - usually burns, tans with difficulty; Type III (scores 17-24) Medium, white to olive - sometimes mild burn, gradually tans to olive. Type IV (scores 25-30) Olive, moderate brown - rarely burns, tans with ease to a moderate brown. Type V (scores over 30) Brown, dark brown - very rarely burns, tans very easily. Type VI Black, very dark brown to black - never burns, tans very easily, deeply pigmented.

"Invasive" refers to a procedure used to collect and/or isolate biological material from a test subject which involves the insertion of an instrument or device through the skin or into a body orifice. Some nonlimiting examples of invasive procedures include biopsies (e.g., punch biopsy, needle biopsy), laser capture microdisection, blood drawn with a syringe, and suction blisters.

"Melanin" means eumelanin and/or pheomelanin.

"Non-invasive" refers to a procedure of collecting and/or isolating biological material from a test subject that does not require insertion of an instrument or device through the skin or a body orifice, does not create a break in the skin, and/or does not directly contact the mucosa. Nonlimiting examples of non-invasive procedures include tape stripping, hair plucking, hair clipping and gentle scrapping.

"PTCA" means pyrrole-2,3,5-tricarboxylic acid and is a derivative of eumalanin.

"Skin" means the outermost protective covering of mammals that is composed of cells such as keratinocytes, fibroblasts and melanocytes. Skin includes an outer epidermal layer and an underlying dermal layer. Skin may also include hair and nails as well as other types of cells commonly associated with skin, such as, for example, myocytes, Merkel cells, Langerhans cells, macrophages, stem cells, sebocytes, nerve cells and adipocytes.

"Skin tone" refers to skin pigmentation and evenness of coloration.

"Tape stripping" means a noninvasive method of collecting biological material involving the use of an adhesive disposed on a substrate. For example, tape stripping may be used to collect biological material from the surface of the skin by contacting a suitable adhesive containing substrate with the surface of the skin such that the biological material adheres to the adhesive.

It is generally recognized that human skin comprises two major layers: the underlying dermal layer and the overlying epidermal layer. The majority of cells in the dermis are fibroblasts, which help form the extra cellular matrix. The cells in the epidermis are primarily keratinocytes and are arranged as layers of differentiated cells. There are four layers of differentiated keratinocytes in the epidermis (five layers in the palms of the hands and soles of the feet). From outermost to innermost, the layers are commonly referred to as: 1) the *stratum corneum* (uppermost layer relative to the underlying dermis); 2) the *stratum lucidum* (only present in palms and soles); 3) the *stratum granulosum* (adjacent the stratum corneum except in palms and soles); 4) the *stratum spinosum*; and 5) the *stratum basale* (also called the stratum germinativum and located nearest the dermis).

In addition to keratinocytes, the epidermis also includes melanocytes, which are generally located in the stratum basale of the epidermis. Melanocytes are responsible for melanogenesis, which involves the production, packaging and distribution of melanin to the keratinocytes. Most epidermal melanin is packaged in melanosomes by melanocytes in the stratum basale (and to a much lesser extent in the stratum spinosum). The melanin containing melanosomes are then distributed to keratinocytes in the basal layer of the epidermis by the dendritic processes.

In skin, melanin acts as an optical and chemical photoprotective filter, which reduces the penetration of the various wavelengths of light into sub-epidermal tissues. Electromagnetic radiation impinging on the human skin can undergo a number of interactions. For example, it can be reflected by the surface of the stratum corneum; it can enter the stratum corneum after a slight change in its direction of travel; it can interact with "melanin dust," which is believed to be a degradation product of melanosomes that persists in the stratum corneum, especially in lighter skinned individuals (e.g., Fitzpatrick I, II, III, or IV skin types), resulting in partial or total absorption; it can traverse deeper layers and experience a possible additional scattering; or it can enter the viable epidermis (i.e., stratum basale) where it encounters melanin packaged in vessicles. In some instances (e.g., in lighter skinned individuals), epidermal melanin has different forms at different sites within the skin that interact differently with radiation. At all these sites, melanin may function as an optical filter to attenuate radiation by scattering. It may also act as a chemical filter through its function as a stable free radical that can absorb compounds produced by photochemical action, which would be toxic or carcinogenic. Thus, the superior photoprotection of highly melanized skin is accomplished by absorption and scattering, which are influenced by the density and distribution of melanosomes within keratinocytes in the basal and parabasal layers of the epidermis and by the presence of specks of melanin dust in the stratum corneum.

The ability to quantify the amount of melanin in the skin has long been useful tool for skin care research and skin disease diagnosis, especially in the area of cosmetic skin care. In particular, there has been a substantial amount of research by the cosmetics industry to identify convenient tools for measuring melanin in skin to develop and evaluate cosmetic products useful for the treatment of undesirable skin tone conditions (e.g., melasma, age spots). In the past, conventional wisdom dictated that in order to accurately determine melanin content in the skin it was necessary to obtain tissue samples from the basal layers of the epidermis where melanogenesis occurs. In other words, it was believed that living cells were needed. While it was known that biological material present in the stratum corneum could be obtained noninvasively (e.g., via tape stripping), it was thought that melanin could not be accurately quantitated from tissue samples obtained only from the stratum corneum, which comprises primarily cornified keratinocytes (i.e., non-living cells). Surprisingly, it has been discovered that PTCA can be obtained from samples of biological material (e.g., skin cells) collected by non-invasive methods such as tape stripping. PTCA obtained in this way can then be quantitated and correlated to the amount of melanin present in the sample. The non-invasive methods disclosed herein overcome some of the drawbacks associated with invasive sampling. For example, tape stripping samples can be readily collected from a large population of people, as opposed to the time consuming and costly collection of biopsy samples.

The present method provides a non-invasive means of collecting biological material from a test subject and obtaining PTCA from the sample (e.g., via digestion). The present method is particularly suitable for use in the field of cosmetics. In some embodiments, the present method may, optionally, include analyzing the PTCA to determine the amount of PTCA present in a sample of biological material. The amount of PTCA may then be correlated to the amount melanin present in the sample.

### SAMPLE COLLECTION

Samples of biological material may be noninvasively obtained from skin present on any part of the body (e.g., face, arm, leg, chest, stomach, back, shoulder). In certain embodiments, a sample may be collected by contacting a target skin surface with an adhesive, and then removing the adhesive along with any biological material (e.g., skin cells) adhered thereto. It may be desirable to provide an adhesive that is safe (i.e., does not cause any serious side effects to a person) and tacky enough to collect a suitable amount of biological material from the skin. It may also be desirable to balance the tackiness of the adhesive so that it is capable of collecting a suitable amount of skin cells, but does not cause an undesirable level of discomfort when contacted with and/or removed from the skin. For example, it may be desirable to use a mild adhesive that can be applied to and removed from even visibly inflamed skin without causing discomfort. Any suitable adhesive known in the art for use on skin may be used herein. Some non-limiting examples of adhesives that may be suitable for use herein include medical-grade adhesives, silicone-based adhesives, polyolefin adhesives, block copolymer adhesives, and pressure-sensitive adhesives. In some embodiments, the adhesive may be applied to or removed from a target skin surface using a suitable applicator (e.g., substrate).

In some embodiments, the adhesive may be disposed on one or both sides of a substrate such that an adhesive-containing side of the substrate can be conveniently contacted with the surface of the skin and subsequently removed. Such configurations are commonly employed in conventional adhesive tapes. The substrate provides a relatively stable support for the adhesive(s) and other optional elements (e.g., release layer or cover sheet) disposed thereon. The adhesive-containing substrate should also include a convenient means for grasping and manipulating the device, especially when contacting the adhesive with skin and subsequently removing it. For example, the adhesive may be disposed only on a portion of the substrate such that an adhesive-free, graspable "tab" is provided. In certain embodiments, the tab may be provided at one or both ends of a rectangular substrate or around all or a portion of the circumference of a circular or elliptical substrate.

The substrate may include one or more layers of a rigid, flexible and/or compressible material with suitable physical properties to resist undesirable deformation and tearing during processing and usage. The substrate may be modified (e.g., surface modified) to include one or more optional compositions to improve certain properties of the substrate (e.g., increased or decreased adhesion, liquid and/or vapor impermeability, increased rigidity, increased tensile strength, improved feel (e.g., warmer, softer, smoother and/or rougher), improved grip-ability and/or a modified appearance (e.g., reflectance, transparency, translucency and/or opacity)). The substrate may be made from a wide range of materials, which are known in the art. Some nonlimiting examples of adhesive containing substrates suitable for use herein include commercially available adhesive tape such as Sebutape™ (acrylic polymer adhesive film) available from CuDerm, Dallas, TX; D-Squame® brand polyacrylate ester adhesive tape available from CuDerm; Durapor™ brand medical tape available from 3M, St. Paul Minnesota; Tegaderm™ 333 brand duct tape available from Nashua tape products1; Scotch® 810 brand tape available from 3M, St. Paul, MN; Diamond™ brand tape available from the Sellotape Company, the Netherlands; and Sentega™ brand polypropylene adhesive tape available from Sentega Eiketten BV, the Netherlands.

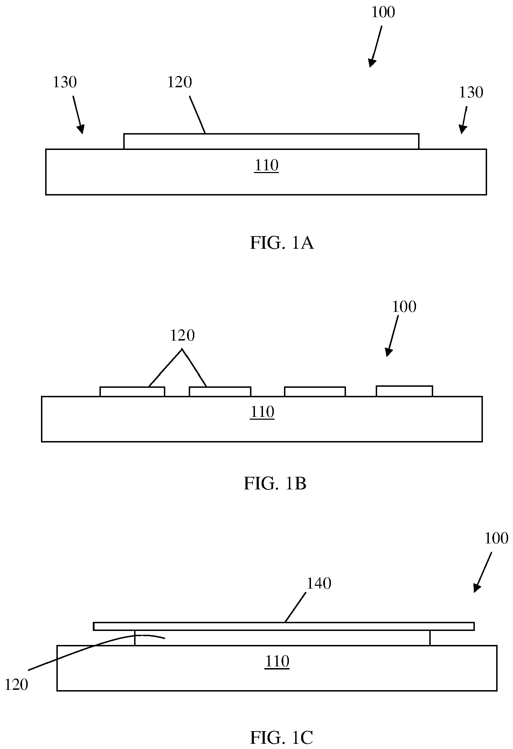

FIGS. 1A, 1B and 1C illustrate examples of a device 100 suitable for collecting samples according to the methods herein. As illustrated in FIG. 1A, the device 100 may include a substrate 110 with a continuous layer of adhesive 120 disposed thereon. The adhesive 120 may be arranged such that an adhesive-free portion 130 is provided at each end of the substrate 110, which can be used as a tab for grasping and manipulating the device 100. FIG. 1B illustrates an example of an embodiment in which the substrate 110 includes a discontinuous layer of adhesive 120. FIG. 1C illustrates the device 100 with a removable cover layer 140, which may help prevent undesirable contamination of the adhesive 120.

In some embodiments, the adhesive may be a film-forming polymer. The film-forming polymer may be applied to a target skin surface in the form of a liquid, semi-solid, paste, gel or the like, and allowed to remain until it forms a suitable film (e.g., once the film-forming material cures or dries). The film may be allowed to remain on the skin for a predetermined amount of time (e.g., between 1 second and 15 minutes, between 10 second and 10 minutes, between 30 seconds and 5 minutes or even about 3 minutes). In this way, biological material on the surface of the target skin area is collected by the film, for example, by adhering to the film or being trapped within the matrix of the film. The film can then be subjected to a suitable process for obtaining and/or analyzing the melanin derivative, such as the method described in more detail below. The characteristics of the film-forming polymer (e.g., adhesion or other chemical or electrostatic interaction) should enable the collection of sufficient biological material to enable quantitation of the melanin derivative present in the sample.

In some embodiments, it may be desirable to identify a particular portion of skin in which to determine melanin content. For example, it may be desirable to determine the melanin content of a portion of skin that is hyperpigemented (e.g., a lentigo or melasma) or hypopigmented (e.g., vitiligo). In some embodiments, it may be desirable to identify an area of skin that includes a skin tone which is different from the skin tone of another portion of skin. In this example, the adhesive may be applied to both portions of skin (i.e., the two areas of skin exhibiting a difference in skin tone) and the amount of melanin determined to be present in each area can be compared to the other area and/or a control. Continuing with this example, the amount of melanin present in a portion of skin commonly exposed to UV radiation (e.g., face, shoulders or forearms) may be compared to an area of skin that is not typically exposed to UV radiation (e.g., inner arm, inner leg, or buttock). In some embodiments, it may be desirable to determine the amount of melanin present in an area of skin commonly associated with discoloration such as the periorbital region of the face.

In some embodiments, it may be desirable to determine a change in melanin of a particular area of skin over time. For example, it may be desirable to measure a change in melanin content of target skin surface that is treated with a personal care product such as skin lightening product. In this example, the melanin content of the target skin surface may be determined at a first time (e.g., just prior to application) and then determined again after a predetermined amount of time has passed (e.g., between 4 hours and 96 hours, between 8 hours and 48 hours, or between 12 hours and 24 hours). The comparative measurements may be taken periodically for a predetermined amount of time (e.g., between 1 week and 6 months) to evaluate the skin tone benefit properties of the personal care product. In some instances, the melanin content may be determined based on a predetermined number of treatments (e.g., daily treatments) with a personal care product (e.g., between 1 and 100 treatments, between 5 and 90 treatments, between 10 and 75 treatments, or between 30 and 60 treatments).

In some embodiments, it may be desirable to determine a change in melanin of a portion of skin due to intrinsic or extrinsic factors (e.g., environmental stressors such as UV radiation, pollution and cigarette smoke and naturally occurring reactive oxygen species). For example, it may be important to monitor changes in melanin over a longer term, as compared to the use of a personal care product, due to the more gradual changes that may be associated with the change in melanin due to such factors. Accordingly, in this example, it may be desirable to determine melanin content two times a week, weekly, 3 times a month, bi-weekly, monthly, or even bimonthly for between one month and 70 years (e.g., between 3 months and one year).

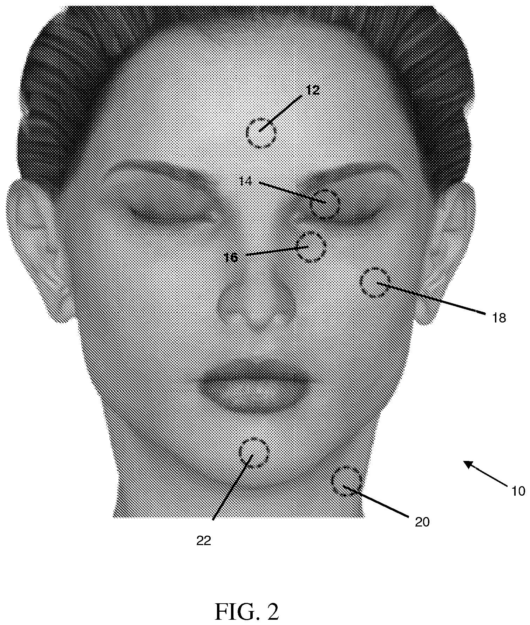

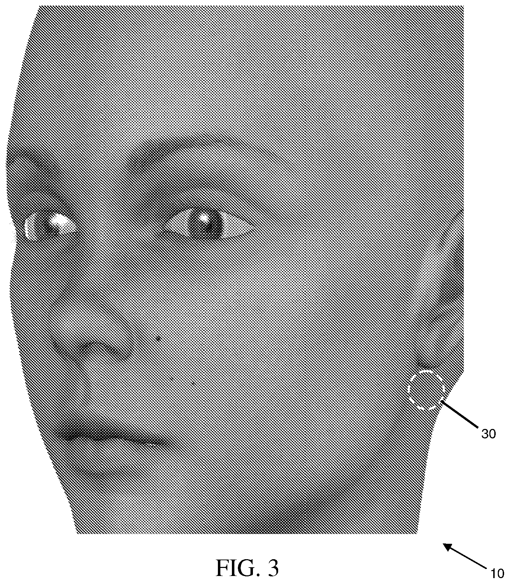

FIGS. 2 and 3 illustrate areas of a human face 10 where it may be desirable to collect samples of biological material for determining melanin content. As illustrated in FIG. 2, it may be desirable to sample the area of skin on or near the forehead 12, the upper eyelid 14, the lower eyelid 16, the cheek 18, the neck 20, the chin 22 and/or behind the ear 30. For example, it may be desirable to determine the melanin content of skin disposed in the upper eyelid 14 and lower eyelid 16 areas of the face 10 in order to determine if a test subject exhibits a particular type of periorbital dyschromia. In another example, it may be desirable to compare the amount of melanin present in the cheek 18 and/or 12 forehead to the amount of melanin present in the chin 22, neck 20 and/or behind the ear 30 to determine if there is a difference in melanin content between two or more of these areas.

When collecting a sample of biological material, the adhesive is contacted with the skin for a sufficient amount of time to collect a sample of biological material. If the adhesive is disposed on a suitable substrate, pressure may be applied to the non-adhesive-containing side of the substrate to help improve the likelihood biological material adhering to the adhesive, but not cause discomfort to the test subject. The adhesive may be left on the skin for at least 1 second (e.g., from 1 second to 5 minutes; from 10 seconds to 2 minutes; from 30 second to 1 minute). After the adhesive has been in contact with the skin for the desired amount of time, the sample (i.e., adhesive plus biological material or adhesive-containing substrate plus biological material when using, for example, a tape strip or tape disc) may be removed and transferred to a suitable storage container for storage and/or later testing, or the sample may be immediately subjected to a process for isolating PTCA, which is described in more detail below. When the adhesive is disposed on a substrate such as a tape strip or tape disk, the size and/or shape of the adhesive-containing substrate may be selected to correspond to the portion of skin to be sampled. For example, a 14 mm diameter D-Squame® brand adhesive disc may be suitable for sampling skin on the face of a person.

### OBTAINING A MELANIN DERIVATIVE

In order to obtain a melanin derivative from a sample of biological material, the sample is subjected to a suitable chemical or physical process for converting a melanin precursor into the desired melanin derivative. PTCA may be obtained from a sample comprising skin cells by chemically digesting the sample with a suitable quantity and concentration of hydrogen peroxide and a suitable base (e.g., sodium hydroxide), which converts melanin into PTCA. However, it is to be appreciated that prior to digesting the sample and/or subjecting the sample to other destructive processes, it may be desirable to conduct non-destructive testing first, such as measuring total protein content. Additionally or alternatively, it may be desirable to include an optional compound in the digest such as an internal standard (e.g., stable isotope or compound of similar structure to the derivative of interest), which can be used to normalize the amount of PTCA to correct for, e.g., in sample loss or analytical instrumental variation. The melanin derivative obtained from the sample may be extracted from the digest by a suitable liquid-liquid extraction procedure as is known in the art. When extracting PTCA from the digest, a water immiscible solvent (e.g., methyl-t-butyl ether, diethylether, chloroform, methylene chloride, hexane or other suitable solvent) that has suitable solubility may be used. In some embodiments, the melanin derivative may be analyzed directly in the digested sample. The digested sample and/or the extracted analyte may be subjected to an analytical analysis technique that provides sufficient specificity and sensitivity for the detection of the desired melanin derivative. For example, a suitable reversed-phase high-performance liquid chromatography ("HPLC") column coupled with a sensitive and selective detection approach such as, but not limited to, tandem mass spectrometry may be particularly suitable for detecting certain melanin derivatives such as PTCA.

### ANALYSIS OF THE MELANIN DERIVATIVE

In certain embodiments, detection and quantitation of the melanin derivative may be done by tandem mass spectrometry (MS/MS) operating under multiple reaction monitoring conditions, as described in more detail in the example below. Prior to any conducting any destructing testing on the sample of biological material (e.g., digestion), it may be desirable to determine the total protein content of the sample. For example, if the sample is obtained via a tape strip, the total protein content of biological material sample may be determined using a suitable scanner (e.g., D-Squame® Scan™ 850 brand scanner available from Heiland Electronic GmbH, Germany or equivalent). In some embodiments, the protein amount determined for a particular sample can be used to normalize the amount of melanin derivative measured on that sample. Normalization may be used to correct for differences in the amount of biological material removed from the skin surface by the tape strips.

The amount of melanin derivative present in a sample may be determined by interpolation from a known standard curve. The amount of PTCA obtained by extracting it from a tape strip digest can be determined by interpolation from a standard curve constructed from known PTCA calibration standards. In this example, the nominal range of quantitation may be from 0.15 to 100 ng of PTCA per tape strip. Continuing with this example, the final result may be reported as the mass of PTCA found from a given tape strip divided by the amount of protein found for the same tape strip (i.e., total mass of analyte (ng or µg) per total weight of protein (µg) per sample).

### EXAMPLE

The following example describes collecting samples from the face of test subject using an adhesive-containing substrate. Of course, it is to be appreciated that the present method may be performed using any suitable means of contacting the adhesive with any area of the body, as desired.

### Example 1

55 female test panelists aged 20 to 45 were selected to participate in the study. The panelists were permitted to wash their face as usual and/or use their normal skin care regimen prior to the test as long as it was at least 8 hours prior to the beginning of the test. On the morning of the test, the panelists were asked not to apply any skin care/make up product on the eyes, and to wash their face with only water. Upon arrival, each panelist was asked to clean their face with an Olay Sensitive® brand wet wipe. The panelist's skin was then allowed to dry. Using clean tweezers, a 14mm diameter D-Squame® brand polyacrylate ester adhesive tape disc was placed on each of the following areas of the face: behind the ear, cheek, chin, forehead, lower eyelid and upper eyelid. To help ensure the tape disc makes good contact with the skin (e.g., no wrinkles), run a gloved finger over the surface of the tape (i.e., the non-adhesive-containing side of the tape). Then, using a medical-grade, cotton-tipped applicator, the non-adhesive containing surface of the tape disc is rubbed five times. Gently remove the tape disc from the test subject using clean tweezers. Place the tape discs in a suitable container (e.g., 48 well culture plate, glass tube, plastic tube) and seal with an appropriate closure. Handle the tape disc carefully to avoid inadvertently contaminating the sample. In particular, avoid contacting the adhesive side of the tape with other surfaces (e.g., sides of the container) or potential sources of contamination. The collected sample and container are then placed on dry ice until transfer to a suitable longer-term storage device (ex. -70°C freezer). The process was repeated for the lower eyelid, cheek forehead and behind the ear of each panelist.

In addition to the foregoing, it is important to follow general sanitary lab practices. For example, suitable gloves should always be worn when handling the tape discs (before and after sample collection). Also, if a tape disc falls onto any surface adhesive side down, the procedure must be started over with a new tape disc on a different portion of the sampling site. Further, the tweezers should be cleaned between samplings and must be completely dry before touching a tape disc.

To analyze the collected samples, the tape discs were removed from storage, allowed to warm to ambient temperature and then each tape disc was placed in The D-Squame® Scan 850A instrument for protein determination. The tape discs are placed adhesive side up in 10-disc cartridges, available in 2 sizes to accommodate either 22 mm or 14 mm (CuDerm, D-101) D-Squame® discs. The D-Squame® Scan 850A emits a beam of light at a wavelength of 850 nm which passes through the D-Squame® disc containing a stratum corneum sample, and measures a decrease in light intensity in a non-destructive manner. Using a wavelength of 850 nm reduces light scattering and ambient light, minimizes errors due to molecular absorption, and prevents thermal denaturation of biomolecules within the stratum corneum. The measurement range of the D-Squame® Scan™ 850A is 0-40% absorption with a resolution of 0.1%. The diameter of the circular slit is 15mm (area = 1.8 cm²) which covers 45% of the area of a standard 22 mm D-Squame® skin sampling disc (available from CuDerm, D-100). The absorption of a blank D-Squame® tape disc is subtracted from the readings to correct for background noise. The SquameScan™ 850A readings are linearly proportional to stratum corneum protein content, and thus can be used to indirectly measure amount of stratum corneum protein present on each D-Squame® disc.

Upon completion of the total protein measurement, carefully remove each tape disc from the storage container using tweezers and place into a suitable digestion container (e.g., 4 mL cryogenic Nalgene® brand tubes). Avoid contacting the tape disc with the wall of the container or any other surface that might contaminate the sample or to which the sample might adhere. The tape disc is then treated with equal volumes of 2 M ammonium hydroxide and 6% hydrogen peroxide, which, in this example, is 0.9 mL each of ammonium hydroxide and 6% hydrogen peroxide. The tubes are capped and vortexed and then placed on a rocker to incubate for three hours at 200 Mot/minute to facilitate the digestion of the melanin. The tubes are removed from the rocker and carefully uncapped to release any pressure that may have built up during incubation. The liquid contents of each tube are transferred to separate 15 mL conical tubes leaving the tape disc in the original container for disposal. Then, 32 µL of 10% sodium bisulfite is added to each conical tube and then the tubes are capped and vortexed. Following vortexing, the caps are removed and an aliquot (50 µL) of a stable-isotope internal standard solution (2 µg/mL) is added to each tube to correct for extraction and instrumental response. The pH of each of the solutions is made acidic by the addition of 1.0 mL of 37% hydrochloric acid. After the pH adjustment, the tubes are capped and vortexed. Next, the caps are removed and 1.0 mL of saturated ammonium sulfate is added along with 2.0 mL of methyl-tert-butyl ether. The tubes are capped, vortexed and centrifuged. After centrifugation, 1.0 mL of the organic layer (where the PTCA and the internal standard are located) of each tube is transferred to separate liquid chromatography vials and dried under nitrogen. The residue in each vial is reconstituted with 0.250 mL of a 10 mM ammonium acetate solution. The vials are capped and vortexed. The samples are analyzed by high-performance liquid chromatography/mass spectrometry/mass spectrometry (HPLC/MS/MS) using Shimadzu pumps and gradient controller and Sciex API 5000 mass spectrometer. It is to be appreciated that any HPLC or mass spectrometry system capable of delivering equivalent performance may be used. The PTCA amount may be normalized based on the total protein content measured previously.

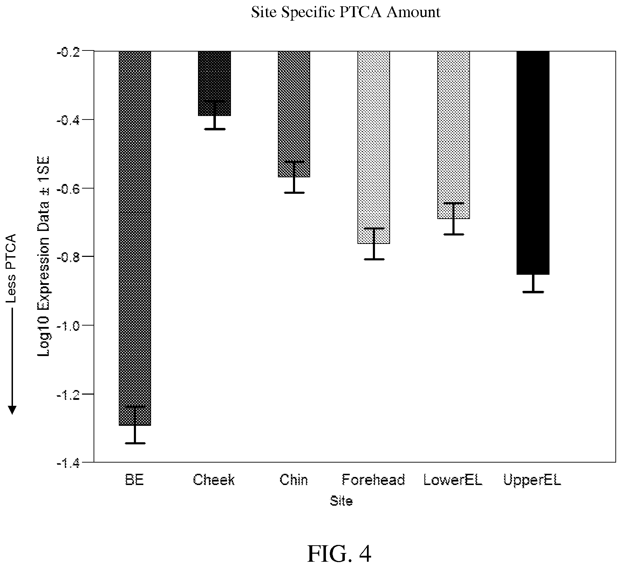

FIG. 4 illustrates the PTCA amount obtained from samples collected at the sites indicated in FIG. 4 and averaged across the 55 test subjects. It is commonly recognized that certain areas of the face are typically darker and include more melanin than other areas of the face due to increased exposure to the sun and a corresponding increase in UV (i.e., ultraviolet radiation) induced melanin production. In particular, the cheeks and chin typically receive the most UV exposure, followed by the eyes and forehead. Not surprisingly, the area of skin found behind the ear typically receives the least amount of UV exposure. As illustrated in FIG. 4, PTCA measured at different areas of skin on the face corresponds to what is typically expected in terms of melanin content. Specifically, tape strip samples taken from the area of skin behind the ear ("BE" in FIG. 4) provided the least amount of PTCA, while tape strip samples taken from cheek provided the most PTCA. The tape strip samples taken from areas around the chin, forehead and eyes (upper eyelid and lower eyelid) provided amounts of PTCA that corresponded to the relative amounts of melanin expected to be present in these areas of the face. Thus, the data illustrated in FIG. 4 demonstrates that the present method can be used to determine the amount of melanin present in a sample of biological material obtained via non-invasive sampling.

### Example 2

This example demonstrates the use of the present method for determining the difference in melanin content between different individuals. Tape strip samples were collected from three locations on five female test subjects identified as having different skin tones from one another. The locations sampled were the upper eyelid, the lower eyelid and behind the ear. Test subject 102 was identified as having a Fitzpatrick IV skin type. Test subject 105 was identified as having a skin type of between Fitzpatrick III and IV. Test subjects 103 and 104 were both identified as having Fitzpatrick skin types of between II and III, but test subject 104 had a slightly darker skin tone than test subject 103. Test subject 101 was identified as having a Fitzpatrick skin type of between I and II. The tape strips samples were collected and analyzed in substantially the same manner as described above in Example 1, except that the PTCA measurement was not normalized based on total protein.

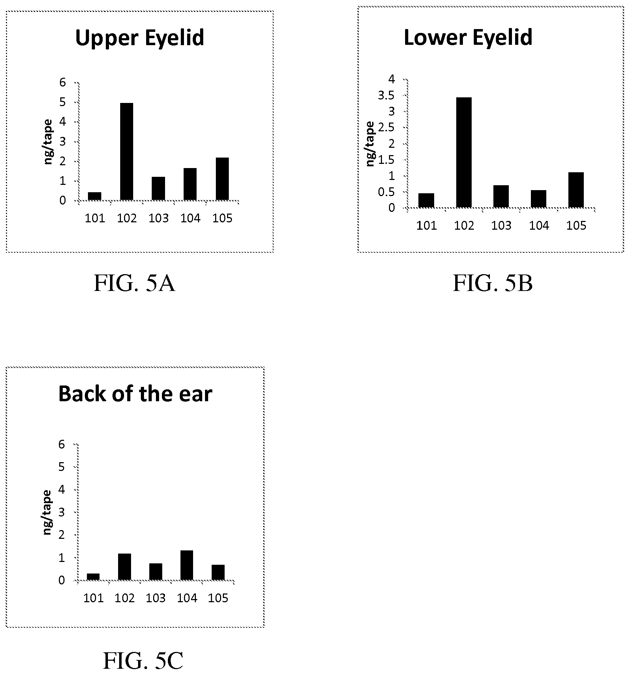

FIG. 5A illustrates the amount of PTCA obtained from the tape strip sample taken from the upper eyelid area of each test subject. As illustrated in FIG. 5A, test subjects 102 and 105 exhibited the highest amounts of PTCA, which is expected since they have darker skin and presumably more melanin present. Similarly, the amount of PTCA obtained from the samples taken from test subjects 101, 103 and 104 corresponded to the relative amounts of melanin that would be expected based on the relative skin tones of the individuals.

FIG. 5B illustrates the amount of PTCA obtained from the tape strip sample taken from the lower eyelid area of each test subject. As illustrated in FIG. 5B, test subjects 102 and 105 again exhibited the highest amounts of PTCA as expected. While the amount of PTCA obtained from the samples taken from test subjects 101, 103 and 104 generally corresponding to what was expected, the differences in PTCA levels between these individuals were somewhat less pronounced as compared to the PTCA levels of the upper eyelid samples. This is likely due to the reduced amount of UV exposure that the lower eyelid area receives compared to the upper eyelid area. The lower eyelid area of the face is subject to shadowing effects from the nose, brow and other facial features. Thus, it is not surprising to see a somewhat smaller difference in the PTCA levels in the area of the face around the lower eyelid.

FIG. 5C illustrates the amount of PTCA obtained from the tape strip sample taken from the area near the back of the ear of each test subject. As illustrated in FIG. 5C, test subject 102 again exhibited the highest amounts of PTCA and test subject 101 exhibited the lowest amounts, which are both expected. However, the sample collected from test subject 104 unexpectedly exhibited higher PTCA levels than test subjects 102 and 105. Upon further investigation, it was realized that test subject 104 had shorter hair than the other test subjects. Test subject 104's hair did not cover the target area of skin near the back of the ear, whereas the other test subjects had long hair that covered the target area of skin near the back of the ear. As a result, the area of skin around the back of the ear of test subject 104 likely received more UV exposure than the other test subjects, which led to increased UV induced melanin production and higher PTCA levels. Thus, the observed PTCA levels actually correspond to what would be generally expected, once this additional variable is factored in. As can be seen from this example, the present method provides sufficient sensitivity to distinguish between the PTCA levels of different individuals even when a visual evaluation may not readily identify a difference in skin tone.

### Example 3

This example demonstrates how the method may be used to determine the differences in PTCA level, and thus melanin content, of different portions of skin of an individual test subject. In this example, a first tape strip samples was collected from a target area of skin on the test subject that included a hyperpigmented portion (i.e., age spot). A second tape strip sample was collected from a nearby portion of skin in the same area of the body that exhibited a normal skin tone (i.e., no hyperpigmentation or hypopigmentation). The tape strips were collected and analyzed according to substantially the same methods as described above in Example 1, except that the samples were not normalized based on total protein.

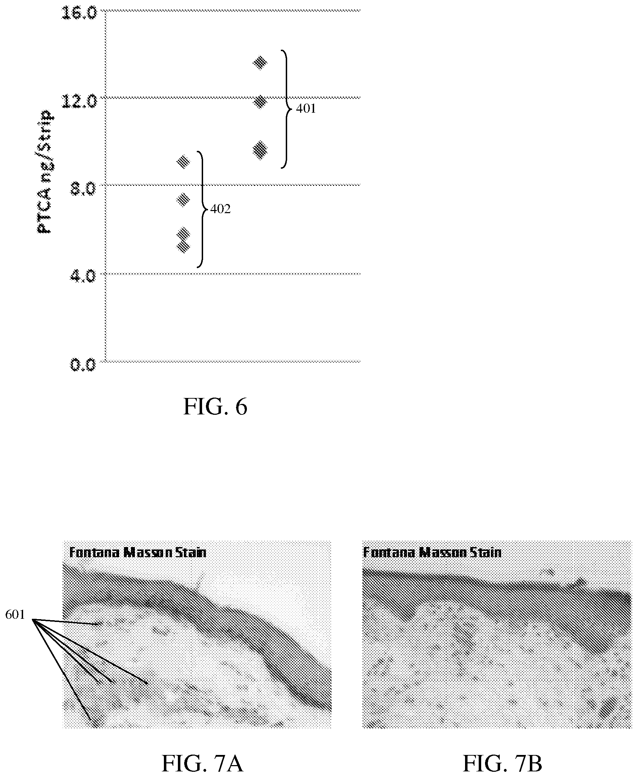

FIG. 6 illustrates the amount of PTCA obtained from the hyperpigmented area 401 and the amount of PTCA obtained from the normal skin tone area 402. As illustrated in FIG. 6, the hyperpigmented sample clearly had more PTCA present, which corresponds to the presence of more melanin. Since it is well recognized that hyperpigmented portions of skin contain more melenin than the nearby normal skin, the results support a conclusion that the present method is suitable for determining the melanin content of different areas of skin on a single individual.

Example 4This example demonstrates that results obtained according to the present method correspond to those observed in a conventional histology analysis for determining melanin content of a sample. Biopsy samples were collected from two test subjects. The samples were processed and stained using Fontana-Masson stain according to conventional methods to quantify the amount of melanin present in the samples. Tape strip samples were also collected from each test subject in the same area of the body as the biopsy. The tape strip samples were collected and analyzed in substantially the same manner as described above in Example 1.

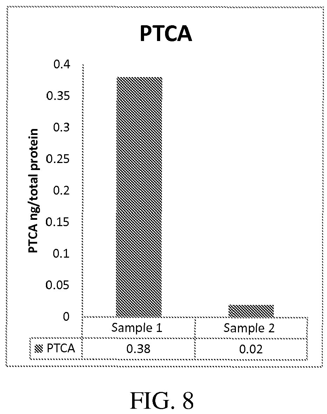

FIGS 7A and 7B show micrographs of the stained samples at 20x magnification. The sample shown in FIG. 7A has significantly more Fontana-Masson positive bodies (shown as small black dots 601) than the sample shown in FIG. 7B, which indicates that the sample in FIG. 7A contains more melanin. FIG. 8 shows the amount of PTCA obtained for each tape strip sample. The sample shown in FIG. 7A is identified as Sample 1 in FIG. 8 and the sample shown in FIG. 7B is identified as Sample 2 in FIG. 8. As can be seen in FIG. 8, the PTCA measured for Sample 1 was significantly higher than Sample 2. Thus, the results of the present method correspond to the results observed in a conventional histology analysis, which further demonstrates that the present method is suitable for quantitating the melanin content of a sample of biological material.

## Claims

1. A non-invasive method of obtaining a melanin derivative, the method comprising:
a. contacting a target skin surface with an adhesive to collect a sample of biological material; and
b. obtaining a melanin derivative from the sample of biological material;
c. using the melanin derivative to develop or evaluate a cosmetic product, wherein the melanin derivative is pyrrole-2,3,5-tricarboxylic acid (PTCA).

2. The method of claim 1, wherein the adhesive is disposed on a substrate.

3. The method of claim 1, wherein the biological material comprises keratinocytes.

4. The method of claim 3, wherein PTCA is obtained from the keratinocytes.

5. The method of any preceding claim, wherein obtaining the melanin derivative comprises digesting the sample of biological material.

6. The method of any preceding claim, wherein obtaining the melanin derivative comprises liquid-liquid extraction.

7. The method of any preceding claim, wherein obtaining the melanin derivative comprises reversed-phase, high-performance liquid chromatography.

8. The method of any preceding claim, wherein obtaining the melanin derivative comprises adding an internal standard to the sample.

9. The method of any preceding claim, wherein the target skin surface includes a hyperpigmented portion or a hypopigmented portion.

10. The method of any preceding claim, wherein the test subject has a skin type classified as Type I, Type II, Type III, or Type IV on the Fitzpatrick Scale.

11. The method of any preceding claim, wherein the adhesive remains in contact with the skin for at least 1 second.

12. The method of any preceding claim, further comprising correlating the amount of melanin derivative to an amount of melanin present in the skin.

13. The method of any preceding claim, further comprising:
e. contacting a second portion of skin with the adhesive to collect a second sample of biological material;
f. obtaining a second melanin derivative from the second sample of biological material;
g. determining an amount of the second melanin derivative obtained from the second sample; and
h. comparing the amount of the first melanin derivative to the amount of the second melanin derivative to determine a relative amount of melanin present in at least one of the first and second portions of skin.

## Patentansprüche

1. Nicht invasives Verfahren zum Erhalten eines Melanin-Derivats, wobei das Verfahren umfasst:
a. Kontaktieren einer Ziel-Hautoberfläche mit einem Haftmittel, um eine Probe biologischen Materials zu erfassen; und
b. Erhalten eines Melanin-Derivats aus der Probe biologischen Materials;
c. Verwenden des Melanin-Derivats zum Entwickeln oder Bewerten eines kosmetischen Produkts, wobei das Melanin-Derivat Pyrrol-2,3,5-Tricarbonsäure (PTCA) ist.

2. Verfahren nach Anspruch 1, wobei das Haftmittel auf einem Substrat angeordnet ist.

3. Verfahren nach Anspruch 1, wobei das biologische Material Keratinozyten umfasst.

4. Verfahren nach Anspruch 3, wobei PTCA von den Keratinozyten erhalten wird.

5. Verfahren nach einem der vorstehenden Ansprüche, wobei das Erhalten des Melanin-Derivats den Verdau der Probe biologischen Materials umfasst.

6. Verfahren nach einem der vorstehenden Ansprüche, wobei das Erhalten des Melanin-Derivats Flüssig-Flüssig-Extraktion umfasst.

7. Verfahren nach einem der vorstehenden Ansprüche, wobei das Erhalten des Melanin-Derivats Umkehrphasen-Hochleistungsflüssigkeits-Chromatografie umfasst.

8. Verfahren nach einem der vorstehenden Ansprüche, wobei das Erhalten des Melanin-Derivats das Hinzufügen eines internen Standards zur Probe umfasst.

9. Verfahren nach einem der vorstehenden Ansprüche, wobei die Ziel-Hautoberfläche einen hyperpigmentierten Abschnitt oder einen hypopigmentierten Abschnitt einschließt.

10. Verfahren nach einem der vorstehenden Ansprüche, wobei das Testsubjekt einen als Typ I, Typ II, Typ III oder Typ IV auf der Fitzpatrick-Skala klassifizierten Hauttyp aufweist.

11. Verfahren nach einem der vorstehenden Ansprüche, wobei das Haftmittel für mindestens 1 Sekunde mit der Haut in Kontakt bleibt.

12. Verfahren nach einem der vorstehenden Ansprüche, ferner umfassend die Korrelation einer Menge von Melanin-Derivat mit einer Menge von in der Haut vorliegendem Melanin.

13. Verfahren nach einem der vorstehenden Ansprüche, weiter umfassend:
e. Kontaktieren eines zweiten Hautabschnitts mit dem Haftmittel, um eine zweite Probe biologischen Materials zu erfassen;
f. Erhalten eines zweiten Melanin-Derivats aus der zweiten Probe biologischen Materials;
g. Bestimmen einer Menge des aus der zweiten Probe erhaltenen zweiten Melanin-Derivats; und
h. Vergleichen der Menge des ersten Melanin-Derivats mit der Menge des zweiten Melanin-Derivats, um eine relative Menge von Melanin zu bestimmen, die in mindestens einem des ersten und des zweiten Hautabschnitts vorliegt.

## Revendications

1. Procédé non invasif d'obtention d'un dérivé de mélanine, le procédé comprenant :
a. la mise en contact d'une surface de peau cible avec un adhésif pour recueillir un échantillon de matériau biologique ; et
b. l'obtention d'un dérivé de mélanine à partir de l'échantillon de matériau biologique ;
c. l'utilisation du dérivé de mélanine pour mettre au point ou évaluer un produit de beauté, dans lequel le dérivé de mélanine est l'acide pyrrole-2,3,5-tricarboxylique (PTCA).

2. Procédé selon la revendication 1, dans lequel l'adhésif est disposé sur un substrat.

3. Procédé selon la revendication 1, dans lequel le matériau biologique comprend des kératinocytes.

4. Procédé selon la revendication 3, dans lequel le PTCA est obtenu à partir des kératinocytes.

5. Procédé selon l'une quelconque des revendications précédentes, dans lequel l'obtention du dérivé de mélanine comprend la digestion de l'échantillon de matériau biologique.

6. Procédé selon l'une quelconque des revendications précédentes, dans lequel l'obtention du dérivé de mélanine comprend une extraction liquide-liquide.

7. Procédé selon l'une quelconque des revendications précédentes, dans lequel l'obtention du dérivé de mélanine comprend une chromatographie liquide à haute performance à inversion de phase.

8. Procédé selon l'une quelconque des revendications précédentes, dans lequel l'obtention du dérivé de mélanine comprend l'ajout d'un étalon interne à l'échantillon.

9. Procédé selon l'une quelconque des revendications précédentes, dans lequel la surface de peau cible inclut une partie hyperpigmentée ou une partie hypopigmentée.

10. Procédé selon l'une quelconque des revendications précédentes, dans lequel le sujet de test a un type de peau classé en tant que Type I, Type II, Type III ou Type IV sur l'échelle de Fitzpatrick.

11. Procédé selon l'une quelconque des revendications précédentes, dans lequel l'adhésif demeure en contact avec la peau pendant au moins 1 seconde.

12. Procédé selon une quelconque revendication précédente, comprenant en outre la corrélation de la quantité de dérivé de mélanine à une quantité de mélanine présente dans la peau.

13. Procédé selon une quelconque revendication précédente, comprenant en outre :
e. la mise en contact d'une deuxième partie de peau avec l'adhésif pour recueillir un deuxième échantillon de matériau biologique ;
f. l'obtention d'un deuxième dérivé de mélanine à partir du deuxième échantillon de matériau biologique ;
g. la détermination d'une quantité du deuxième dérivé de mélanine obtenu à partir du deuxième échantillon ; et
h. la comparaison de la quantité du premier dérivé de mélanine à la quantité du deuxième dérivé de mélanine pour déterminer une quantité relative de mélanine présente dans au moins l'une des première et deuxième parties de peau.
